(19) Europäisches Patentamt
European Patent Office
Office européen des brevets

(11) Publication number: 0 045 200 B1

(12) EUROPEAN PATENT SPECIFICATION

(45) Date of publication of patent specification: 02.03.88

(21) Application number: 81303416.2

(22) Date of filing: 24.07.81

(51) Int. Cl.⁴: A 61 K 31/415, A 61 K 31/425, A 61 K 31/47, A 61 K 31/445, A 61 K 31/44, C 07 D 235/28, C 07 D 401/12, C 07 D 403/12, C 07 D 417/12

# (54) Benzimidazoles and their pharmaceutical use.

(30) Priority: 28.07.80 US 173233

(43) Date of publication of application: 03.02.82 Bulletin 82/05

(45) Publication of the grant of the patent: 02.03.88 Bulletin 88/09

(84) Designated Contracting States: BE CH DE FR GB IT LI NL SE

(56) References cited:
EP-A-0 005 129
DE-A-2 548 340
DE-A-2 840 591
FR-A-2 319 346
GB-A-1 152 814
GB-A-1 525 958
US-A-4 045 563
US-A-4 045 564

(73) Proprietor: THE UPJOHN COMPANY
301 Henrietta Street
Kalamazoo, Michigan 49001 (US)

(72) Inventor: Ruwart, Mary Jean
3664 Cardinal
Kalamazoo Michigan (US)

(74) Representative: Perry, Robert Edward et al
GILL JENNINGS & EVERY 53-64 Chancery Lane
London WC2A 1HN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to a new use for known benzimidazoles, i.e. in the prevention or treatment of gastrointestinal inflammatory diseases.

Benzimidazoles of formula I, and their utility as gastric anti-secretory agents, making them useful in the treatment of gastric ulcers, are disclosed in US—A—4045563 and EP—A—0005129. Some at least of these benzimidazoles inhibit gastric secretion by direct action on the acid-secreting parietal cells of the stomach, specifically via inhibition of potassium ion-dependent ATPase. See, for example, Olbe *et al*, Scand. J. Gastroenterology *14* (1979 Sup. 55) 131—135.

Other known gastric anti-secretory agents are the prostaglandins disclosed in US—A—3903297 and histamine $H_2$ receptor antagonists such as metiamide and, most particularly, cimetidine.

Certain prostaglandins, including gastric anti-secretory prostaglandins, have a cytoprotective effect on the gastrointestinal tract. This effect is manifest in the ability of such compounds to treat or prevent non-traumatically-induced, non-neoplastic inflammatory diseases of the gastrointestinal tract. Cytoprotective prostaglandins are described in US—A—4081553, US—A—4083998 and US—A—4097603.

Further, it appears that the endogenous production of prostaglandins by cells of the gastrointestinal tract represents at least a part of a natural cytoprotective mechanism. For example, when a non-steroidal anti-inflammatory compound is administered to a mammal, one effect is the inhibition of prostaglandin biosynthesis in the gastrointestinal tract, resulting in gastricitis and gastrointestinal blood loss, but this effect is alleviated when a prostaglandin is administered together with the anti-inflammatory compound.

Three classes of cytoprotective action are known to affect beneficially gastrointestinal inflammatory diseases. There is the direct cytoprotective effect exhibited by prostaglandins, the inhibitory effect on the consequences of prostaglandin synthetase inhibition, and the inhibitory effect on the consequences of gastric acid secretion. Only the first of these effects can unconditionally be referred to as "cytoprotective", but the second effect is also commonly known as "cytoprotective". Accordingly, the terms "cytoprotective effect", "cytoprotective action" and "cytoprotection" as used herein will refer both to direct cytoprotection (see US—A—4081553, US—A—4083998 and US—A—4097603) and the inhibition of non-steroidal anti-inflammatory compound-induced effects (see US—A—3781429 and US—A—3927213).

Although prostaglandins may be both cytoprotective and inhibit gastric secretion, not all gastric anti-secretory agents exhibit appreciable cytoprotective effects. For example, Robert, Gastroenterology *76* (May 1979) 1227, and references cited therein, show that any apparent cytoprotective effects of cimetidine are related to anti-secretory properties. Further, Kauffman *et al*, Proc. Soc. Exp. Biol. Med. *161* (1979) 512—4, disclose that cimetidine does not inhibit indomethacin-induced small bowel ulceration in the rat.

Further, antacids which effectively neutralise gastric acid are not cytoprotective.

According to the present invention, a compound which is used for the manufacture of a medicament for the cytoprotective protection of a non-gastric acid-induced, non-traumatically-induced, non-neoplastic gastrointestinal inflammatory disease is a compound of the formula

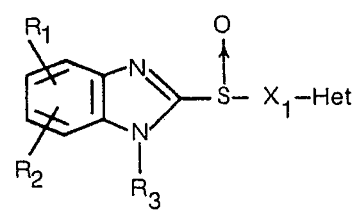

I

wherein either $R_1$ and $R_2$ are independently selected from H, $C_{1-4}$ alkyl, F, Cl, Br, I, CN, COOH, carboxy($C_{1-4}$ alkyl), ($C_{1-4}$ alkoxy)carbonyl, ($C_{1-4}$ alkoxy)carbonyl($C_{1-4}$ alkyl), carbamoyl, carbamoyloxy, OH, $C_{1-5}$ alkoxy, $C_{1-7}$ hydroxyalkyl, $CF_3$ or ($C_{1-4}$ alkyl)carbonyl;

$R_3$ is H, $C_{1-4}$ alkyl, ($C_{1-3}$ alkyl)carbonyl, ($C_{1-4}$ alkoxy)carbonyl, carbamoyl, ($C_{1-4}$ alkyl)carbamoyl, di($C_{1-4}$ alkyl)carbamoyl, ($C_{1-3}$ alkyl)carbonylmethyl, ($C_{1-3}$ alkoxy)carbonylmethyl or $C_{1-4}$ alkylsulfonyl;

$X_1$ is $C_{1-4}$ alkylene; and

Het is optionally substituted imidazolyl, imidazolinyl, benzimidazolyl, thiazolyl, thiazolinyl, quinolyl, piperidyl or pyridyl, in which there may be up to 3 substituents selected from $C_{1-4}$ alkyl, F, Cl, Br and I; or

$R_1$ and $R_2$ are independently selected from H; $C_{1-4}$ alkyl, F, Cl, Br, I, methoxycarbonyl, ethoxycarbonyl, $C_{1-4}$ alkoxy and ($C_{1-4}$ alkyl)carbonyl;

$R_3$ is hydrogen; and

—$X_1$-Het is taken together and is ring-substituted 1-phenyl-$C_{1-3}$ alkyl, in which there are 1, 2 or 3 substituents independently selected from methyl, methoxy, ethoxy, methoxyethoxy and ethoxyethoxy, with the proviso that there is not a single methyl substituent;

or a pharmacologically acceptable salt thereof.

Preferred compounds for use in the invention are

2-[(3,5-dimethyl-4-methoxy-2-pyridyl)methylsulfinyl]benzimidazole,

2-(2-pyridylmethylsulfinyl)benzimidazole,
2-[(4-chloro-2-pyridyl)methylsulfinyl]benzimidazole,
2-[(5-methyl-2-pyridyl)methylsulfinyl]benzimidazole,
2-[1-(2-pyridyl)ethylsulfinyl]benzimidazole,
2-[1-(2-pyridyl)propylsulfinyl]benzimidazole,
2-[(3-methyl-2-pyridyl)methylsulfinyl]benzimidazole,
2-[(5-ethyl-2-pyridyl)methylsulfinyl]benzimidazole,
2-[1-(2-pyridyl)-2-methylpropylsulfinyl]benzimidazole,
2-(2-pyridylmethylsulfinyl)-N-methylbenzimidazole,
2-(2-pyridylmethylsulfinyl)-N-acetylbenzimidazole,
2-(2-pyridylmethylsulfinyl)-N-methoxycarbonylbenzimidazole,
2-(2-pyridylmethylsulfinyl)-N-carbamoylbenzimidazole,
2-(2-pyridylmethylsulfinyl)-N-(methylcarbamoyl)benzimidazole,
2-(2-pyridylmethylsulfinyl)-N-(acetylmethyl)benzimidazole,
2-(2-pyridylmethylsulfinyl)-N-(ethoxycarbonylmethyl)benzimidazole,
2-(2-pyridylmethylsulfinyl)-N-(methylsulfonyl)benzimidazole,
2-[(3,5-dimethyl-2-pyridyl)methylsulfinyl]-4,6-dimethylbenzimidazole,
2-[(4-methoxy-2-pyridyl)methylsulfinyl]-4,6-dimethylbenzimidazole,
2-(2-pyridylmethylsulfinyl)-4,6-dimethylbenzimidazole,
2-(2-pyridylmethylsulfinyl)-4-methyl-6-chlorobenzimidazole,
2-(2-pyridylmethylsulfinyl)-4-methylbenzimidazole,
2-[(3,5-dimethyl-4-methoxy-2-pyridyl)methylsulfinyl]-5-methylbenzimidazole,
2-(2-pyridylmethylsulfinyl)-5-methylbenzimidazole,
2-[(4-methyl-2-pyridyl)methylsulfinyl]-5-methylbenzimidazole,
2-[(5-methyl-2-pyridyl)methylsulfinyl]-5-methylbenzimidazole,
2-[(5-ethyl-2-pyridyl)methylsulfinyl]-5-methylbenzimidazole,
2-[1-(2-pyridyl)propylsulfinyl]-5-methylbenzimidazole,
2-(2-pyridylmethylsulfinyl)-5-ethylbenzimidazole,
2-[1-(2-pyridyl)ethylsulfinyl]-5-ethylbenzimidazole,
2-[1-(2-pyridyl)propylsulfinyl]-5-ethylbenzimidazole,
2-(2-pyridylmethylsulfinyl)-5-isopropylbenzimidazole,
2-(2-pyridylmethylsulfinyl)-5-(t-butyl)benzimidazole,
2-(2-pyridylmethylsulfinyl)-5-(n-propyl)benzimidazole,
2-[1-(2-pyridyl)ethylsulfinyl]-5,6-dimethylbenzimidazole,
2-(2-pyridylmethylsulfinyl)-5,6-dimethylbenzimidazole,
2-[(3,4-dimethyl-2-pyridyl)methylsulfinyl]-5-acetyl-6-methylbenzimidazole,
2-[(4,5-dimethyl-2-pyridyl)methylsulfinyl]-5-acetyl-6-methylbenzimidazole,
2-[(3,5-dimethyl-2-pyridyl)methylsulfinyl]-5-acetyl-6-methylbenzimidazole,
2-[(3,4,5-trimethyl-2-pyridyl)methylsulfinyl]-5-acetyl-6-methylbenzimidazole,
2-[(4-methoxy-2-pyridyl)methylsulfinyl]-5-acetyl-6-methylbenzimidazole,
2-[(3,5-dimethyl-4-methoxy-2-pyridyl)methylsulfinyl]-5-acetyl-6-methylbenzimidazole,
2-[(3,5-dimethyl-4-methoxy-2-pyridyl)methylsulfinyl]-5-acetylbenzimidazole,
2-(2-pyridylmethylsulfinyl)-5-acetylbenzimidazole,
2-[(4,5-dimethyl-2-pyridyl)methylsulfinyl]-5-methoxycarbonylbenzimidazole,
2-[(3,5-dimethyl-2-pyridyl)methylsulfinyl]-5-methoxycarbonylbenzimidazole,
2-[(3,5-dimethyl-4-methoxy-2-pyridyl)methylsulfinyl]-5-methoxycarbonylbenzimidazole,
2-(2-pyridylmethylsulfinyl)benzimidazole-5-carboxylic acid ethyl ester,
2-[(4,5-dimethyl-2-pyridyl)methylsulfinyl]-5-methoxycarbonyl-6-methylbenzimidazole,
2-[(3,4-dimethyl-2-pyridyl)methylsulfinyl]-5-methoxycarbonyl-6-methylbenzimidazole,
2-[(3,5-dimethyl-2-pyridyl)methylsulfinyl]-5-methoxycarbonyl-6-methylbenzimidazole,
2-[(3,4,5-trimethyl-2-pyridyl)methylsulfinyl]-5-methoxycarbonyl-6-methylbenzimidazole,
2-[(4-methoxy-2-pyridyl)methylsulfinyl]-5-methoxycarbonyl-6-methylbenzimidazole,
2-[(4-ethoxy-2-pyridyl)methylsulfinyl]-5-methoxycarbonyl-6-methylbenzimidazole,
2-[(3-methyl-4-methoxy-2-pyridyl)methylsulfinyl]-5-methoxycarbonyl-6-methylbenzimidazole,
2-[(3,5-dimethyl-4-methoxy-2-pyridyl)methylsulfinyl]-5-methoxycarbonyl-6-methylbenzimidazole,
2-[(4-methoxy-5-methyl-2-pyridyl)methylsulfinyl]-5-methoxycarbonyl-6-methylbenzimidazole,
2-(2-pyridylmethylsulfinyl)-6-chlorobenzimidazole,
2-[(3,5-dimethyl-4-methoxy-2-pyridyl)methylsulfinyl]-5-chlorobenzimidazole,
2-(2-pyridylmethylsulfinyl)-5-chlorobenzimidazole,
2-[1-(2-pyridyl)propylsulfinyl]-5-chlorobenzimidazole,
2-(2-pyridylmethylsulfinyl)-4-chlorobenzimidazole,
2-[(4-methoxy-5-methyl-2-pyridyl)methylsulfinyl]-5-methoxybenzimidazole,
2-[(3,5-dimethyl-4-methoxy-2-pyridyl)methylsulfinyl]-5-methoxybenzimidazole,
2-(2-pyridylmethylsulfinyl)-5-methoxybenzimidazole,
2-(2-pyridylmethylsulfinyl)-5-hydroxybenzimidazole,

2-(2-pyridylmethylsulfinyl)benzimidazole-5-carboxylic acid,
2-[1-(2-pyridyl)ethylsulfinyl]-5-cyanobenzimidazole,
2-[1-(2-pyridyl)ethylsulfinyl]-5-trifluoromethylbenzimidazole,
2-[1-(2-pyridyl)propylsulfinyl]-5-cyanobenzimidazole, and
2-[1-(2-pyridyl)propylsulfinyl]-5-trifluoromethylbenzimidazole.

The term "non-gastric acid-induced, non-traumatically-induced, non-neoplastic gastrointestinal inflammatory disease" is used herein in its ordinary and conventional sense, such as in US—A—4083998.

Mammals suffering from these inflammatory diseases are readily diagnosed by an attending physician or veterinarian of ordinary skill in the art. Mammals particularly susceptible to the development of these diseases are those mammals whose environment or medial history provides a known predisposition to gastrointestinal inflammatory disease. For example, mammals especially susceptible to gastric inflammatory diseases include those:

(1) with a history of multiple episodes of gastric or duodenal ulceration,

(2) with a history of chronic and excesssive ethanol consumption,

(3) with a recent acute exposure to a cytodestructive dose of ionizing radiation,

(4) with an acute or chronic ingestive exposure to cytodestructive chemical agents, including cytodestructive chemical agents, or

(5) with a recent exposure to pathogens capable of producing cytodestructive conditions. Similarly, those mammals with a history of colitis, especially ulcerative colitis, are likewise known in the art to be particularly susceptible to recurrence of this gastrointestinal inflammatory disease.

Mammals to whom the cytoprotective heterocyclylalkylsulfinylbenzimidazoles are administered are particularly and especially humans, although other valuable domestic animals and experimental animals are also included within the scope of the present invention.

In accordance with the novel methods employing these cytoprotective heterocyclylalkylsulfinylbenzimidazoles, the present invention provides for oral administration. Accordingly, oral administration as described in European Published Patent Application 0 005 152 and United States Patent 4,045, 563 is conveniently employed.

Appropriate oral pharmaceutical compositions are formulated for use in accordance with the present invention. Such pharmaceutical compositions are employed as described in European Published Patent Application 0 005 152 and United States Patent 4,045,563, except that in place of an antisecretory amount of the heterocyclylalkylsulfinylbenzimidazole, a cytoprotective amount, i.e., an amount effective to treat or prevent the non-traumatically-induced, non-neoplastic gastrointestinal inflammatory disease, is employed in the composition.

Particularly and especially, the compositions employed in accordance with the present invention are in a unit dosage form, as that term is employed in the art, and contain in the unit dosage an amount of the cytoprotective heterocyclylalkylsulfinylbenzimidazole which is both effective to treat or prevent the gastric inflammatory disease and less than the gastric antisecretory $ED_{50}$ (dose effective to inhibit 50% of basal acid secretion) of the cytoprotective heterocyclylalkylsulfinylbenzamidizole. Hence these compositions, which by definition provide insubstantial gastric antisecretory effects, are nonetheless surprisingly and unexpectedly effective as cytoprotective pharmaceutical compositions.

The amount of the cytoprotective heterocyclylalkylsulfinylbenzimidazole effective to treat or prevent the gastrointestinal inflammatory diseases is an amount which varies according to the mammal being treated, the severity of the disease, the route of administration selected, and the particular heterocyclylalkylsulfinylbenzimidazole being employed. Ordinarily, the relative potencies of these cytoprotective heterocyclylalkylsulfinylbenzimidazoles are determined by tests in standard laboratory animals described in Example 1 and employed in accordance with the present method in proportion to their relative potencies. Accordingly, such determinations are readily within the skill of pharmacologists in the art.

These cytoprotective heterocyclylalkylsulfinylbenzimidazoles are accordingly adminisered one to 6 times daily at dosages between from about 0.1 μg to about 100 mg/kg/day orally.

Prevention of the gastrointestinal inflammatory diseases results in the reduction of the incidence and severity of ulceration ordinarily produced by the disease, including total prevention of the inflammatory process. Treatment results in accelerated and more complete and satisfactory healing of ulcers and other manifestations of the inflammatory process.

In the following specific description, Examples 1 to 3 illustrate the utility of the invention, using timoprazole, i.e. 2-(2-pyridylmethylsulfinyl)benzimidazole, and Example 4 illustrates a composition for use in the invention.

## Example 1

Effect of timoprazole on ethanol-induced gastric lesions

A. Four groups of rats, standard laboratory animals for determining cytoprotective effects of pharmacological agents, are fasted for 24 hr and then treated orally with (a) timoprazole (in vehicle), (b) cimetidine (in vehicle), or (c) vehicle (10% Emulphor, 10% EtOH, 80% water). Thirty min later, the rats are challenged with an oral 1 ml dose of 80% aqueous ethanol, a standard agent for inducing gastric cytodestruction. One hr later, the rats are sacrificed (carbon dioxide asphyxiation) and the gastric tissues

examined. Rats treated with timoprazole at 0.3, 1, 3, or 10 mg/kg demonstrate no gastric ulceration. Rats treated with a dose of 0.3 mg/kg show traces of mild ulceration. In contrast, rats treated with vehicle only or with cimetidine at 0.3, 1.0, 3.0 or 10.0 mg/kg all demonstrate gastric ulcers, although a slight decrease in the number of ulcers is noted in the cimetidine-treated animals. The $ED_{50}$ of timoprazole for gastric acid secretion inhibition is at least ten times greater than the lowest cytoprotective dose observed in this experiment.

B. A further experiment is undertaken to rule out gastric emptying as a factor in the cytoprotection observed in part A above. In this experiment timoprazole was given orally at 0.01, 0.1, or 1.0 mg/kg to groups of rats and a further group of rats is treated with vehicle (same as in part A) only. As above, the rats are fasted for 24 hr prior to treatment. Groups of rats are then sacrificed 5, 15, and 30 min after timoprazole ingestion. No significant changes in gastric volume between rats treated with vehicle and rats treated with timoprazole is observed at any of the three sacrifice times. Accordingly, fluid accumulation in the stomach is not a factor in the cytoprotection observed in part A.

C. A further experiment is performed to determine the lowest dose of timoprazole at which 50% of the animals treated exhibit no gastric ulceration ($ED_{50}$), employing a vehicle with less "mild irritant" effects than in part A. Groups of rats are fasted for 24 hr and then treated orally or subcutaneously with timoprazole at 1, 3, or 10 mg/kg in vehicle (5% EtOH, 1% Emulphor in water) at 30 min before ethanol challenge. The rats are sacrificed one hour after timoprazole administration and the extent of gastric ulceration determined. Subcutaneous administration of timoprazole did not significantly reduce the number of ulcers compared to control (vehicle-treated) rats. Oral administration produced a significant reduction in ulceration at all dosages tested and the $ED_{50}$ is determined to be below 1 mg/kg.

D. Because mild gastric irritants, e.g., 20% ethanol, are known to stimulate endogenous prostaglandin production, with consequent cytoprotection effects being observed, a further experiment is undertaken to determine whether timoprazole acts as a mild irritant (i.e., by stimulating endogenous cytoprotective prostaglandin production), or exerts a directly cytoprotective effect. In this experiment, rats are treated with timoprazole (5 mg/kg orally), but in each group of rats half the animals are pretreated with a NOSAC, indomethacin, 1 hr prior to treatment with timoprazole. One hour after ethanol challenge, the rats are sacrificed. However, no significant difference is observed between rats receiving indomethicin and those not receiving indomethicin. Accordingly, the cytoprotective effects of timoprazole are not mediated by endogenous prostaglandin production.

Example 2

Cytoprotective effect of timoprazole against thermally induced gastric ulceration.

Following the procedure of Example 1A, but employing boiling water (greater than 85—95°C) in place of 80% ethanol, there is obtained a uniform reduction in the incidence and severity of ulcers in timoprazole-treated rats (0.3, 1.0, 3.0, and 10.0 mg/kg) as compared to rats receiving vehicle only. Using the ulcer incidence (ulcers/stomach) scoring system of Robert (U.S. Patent 4,097,603, column 4, lines 41—53), the control group exhibits a score of 8.0, while the treated groups exhibit scores of 7.6 (0.3 mg/kg), 5.0 (1.0 mg/kg), 3.9 (3.0 mg/kg), and 4.0 (10.0 mg/kg).

Example 3

Cytoprotective effect of timoprazole against acid-induced gastric ulceration

Following the procedure of Example 1A but employing 0.6N HCl in place of 80% ethanol, there is obtained a uniform reduction in the incidence and severity of ulcers in timoprazole-treated rats (1, 3, 10 and 30 mg/kg) as compared to rats receiving vehicle only. Using the ulcer incidence (ulcers/stomach) scoring system of Robert (US Patent 4 097 603, column 4, lines 41—53), the control group exhibits a score of 9.6, while the treated groups exhibit scores of 6.7 (1 mg/kg), 2.2 (3 mg/kg), 1.5 (10 mg/kg) and 1.2 (30 mg/kg).

Example 4

2-[2-pyridylmethylsulfinyl]benzimidazole HCl (100 g) is mixed with lactose (527.4 g), potato starch (509.1 g), and colloidal silicic acid (96 g). The mixture is moistened with 10% solution of gelatin and is ground through a 12-mesh sieve. After drying, potato starch (480 g), talc (150 g) and magnesium stearate (15 g) are added and the mixture thus obtained is pressed into 10,000 tablets, with each containing 10 mg of active substance. Tablets containing 2.5 mg of active substance are similarly prepared by using 25 g of 2-[2-pyridylmethylsulfinyl]benzimidazole.HCl in the formulation.

**Claims**

1. Use of a compound for the manufacture of a medicament for the cytoprotective protection of a non-gastric acid-induced, non-traumatically-induced, non-neoplaastic gastrointestinal inflammatory disease, in which the compound has the formula

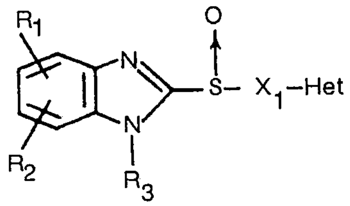

I

wherein either $R_1$ and $R_2$ are independently selected from H, $C_{1-4}$ alkyl, F, Cl, Br, I, CN, COOH, carboxy($C_{1-4}$ alkyl), ($C_{1-4}$ alkoxy)carbonyl, ($C_{1-4}$ alkoxy)carbonyl($C_{1-4}$ alkyl), carbamoyl, carbamoyloxy, OH, $C_{1-5}$ alkoxy, $C_{1-7}$ hydroxyalkyl, $CF_3$ or ($C_{1-4}$ alkyl)carbonyl;

$R_3$ is H, $C_{1-4}$ alkyl, ($C_{1-3}$ alkyl)carbonyl, ($C_{1-4}$ alkoxy)carbonyl, carbamoyl, ($C_{1-4}$ alkyl)carbamoyl, di($C_{1-4}$ alkyl)carbamoyl, ($C_{1-3}$ alkyl)carbonylmethyl, ($C_{1-3}$ alkoxy)carbonylmethyl or $C_{1-4}$ alkylsulfonyl;

$X_1$ is $C_{1-4}$ alkylene; and

Het is optionally substituted imidazolyl, imidazolinyl, benzimidazolyl, thiazolyl, thiazolinyl, quinolyl, piperidyl or pyridyl, in which there may be up to 3 substituents selected from $C_{1-4}$ alkyl, F, Cl, Br and I; or

$R_1$ and $R_2$ are independently selected from H, $C_{1-4}$ alkyl, F, Cl, Br, I, methoxycarbonyl, ethoxycarbonyl, $C_{1-4}$ alkoxy and ($C_{1-4}$ alkyl)carbonyl;

$R_3$ is hydrogen; and

—$X_1$-Het is taken together and is ring-substituted 1-phenyl-$C_{1-3}$ alkyl, in which there are 1, 2 or 3 substituents independently selected from methyl, methoxy, ethoxy, methoxyethoxy and ethoxyethoxy, with the proviso that there is not a single methyl substituent;

or a pharmacologically acceptable salt thereof.

2. A use according to claim 1, in which the compound is selected from
2-[(3,5-dimethyl-4-methoxy-2-pyridyl)methylsulfinyl]benzimidazole,
2-(2-pyridylmethylsulfinyl)benzimidazole,
2-[(4-chloro-2-pyridyl)methylsulfinyl]benzimidazole,
2-[(5-methyl-2-pyridyl)methylsulfinyl]benzimidazole,
2-[1-(2-pyridyl)ethylsulfinyl]benzimidazole,
2-[1-(2-pyridyl)propylsulfinyl]benzimidazole,
2-[(3-methyl-2-pyridyl)methylsulfinyl]benzimidazole,
2-[(5-ethyl-2-pyridyl)methylsulfinyl]benzimidazole, and
2-[1-(2-pyridyl)-2-methylpropylsulfinyl]benzimidazole.

3. A use according to claim 1, in which the compound is selected from
2-(2-pyridylmethylsulfinyl)-N-methylbenzimidazole,
2-(2-pyridylmethylsulfinyl)-N-acetylbenzimidazole,
2-(2-pyridylmethylsulfinyl)-N-methoxycarbonylbenzimidazole,
2-(2-pyridylmethylsulfinyl)-N-carbamoylbenzimidazole,
2-(2-pyridylmethylsulfinyl)-N-(methylcarbamoyl)benzimidazole,
2-(2-pyridylmethylsulfinyl)-N-(acetylmethyl)benzimidazole,
2-(2-pyridylmethylsulfinyl)-N-(ethoxycarbonylmethyl)benzimidazole, and
2-(2-pyridylmethylsulfinyl)-N-(methylsulfonyl)benzimidazole.

4. A use according to claim 1, in which the compound is selected from
2-[(3,5-dimethyl-2-pyridyl)methylsulfinyl]-4,6-dimethyl-benzimidazole,
2-[(4-methoxy-2-pyridyl)methylsulfinyl]-4,6-dimethyl-benzimidazole,
2-(2-pyridylmethylsulfinyl)-4,6-dimethylbenzimidazole,
2-(2-pyridylmethylsulfinyl)-4-methyl-6-chlorobenzimidazole, and
2-(2-pyridylmethylsulfinyl)-4-methylbenzimidazole.

5. A use according to claim 1, in which the compound is selected from
2-[(3,5-dimethyl-4-methoxy-2-pyridyl)methylsulfinyl]-5-methylbenzimidazole,
2-(2-pyridylmethylsulfinyl)-5-methylbenzimidazole,
2-[(4-methyl-2-pyridyl)methylsulfinyl]-5-methylbenzimidazole,
2-[(5-methyl-2-pyridyl)methylsulfinyl]-5-methylbenzimidazole,
2-[(5-ethyl-2-pyridyl)methylsulfinyl]-5-methylbenzimidazole,
2-[1-(2-pyridyl)propylsulfinyl]-5-methylbenzimidazole,
2-(2-pyridylmethylsulfinyl)-5-ethylbenzimidazole,
2-[1-(2-pyridyl)ethylsulfinyl]-5-ethylbenzimidazole,
2-[1-(2-pyridyl)propylsulfinyl]-5-ethylbenzimidazole,
2-(2-pyridylmethylsulfinyl)-5-isopropylbenzimidazole,
2-(2-pyridylmethylsulfinyl)-5-(t-butyl)benzimidazole,
2-(2-pyridylmethylsulfinyl)-5-(n-propyl)benzimidazole,
2-[1-(2-pyridyl)ethylsulfinyl]-5,6-dimethylbenzimidazole, and
2-(2-pyridylmethylsulfinyl)-5,6-dimethylbenzimidazole.

6. A use according to claim 1, in which the compound is selected from 2-[(3,4-dimethyl-2-pyridyl)methylsulfinyl]-5-acetyl-6-methylbenzimidazole, 2-[(4,5-dimethyl-2-pyridyl)methylsulfinyl]-5-acetyl-6-methylbenzimidazole, 2-[(3,5-dimethyl-2-pyridyl)methylsulfinyl]-5-acetyl-6-methylbenzimidazole, 2-[(3,4,5-trimethyl-2-pyridyl)methylsulfinyl]-5-acetyl-6-methylbenzimidazole, 2-[(4-methoxy-2-pyridyl)methylsulfinyl]-5-acetyl-6-methylbenzimidazole, 2-[(3,5-dimethyl-4-methoxy-2-pyridyl)methylsulfinyl]-5-acetyl-6-methylbenzimidazole, 2-[(3,5-dimethyl-4-methoxy-2-pyridyl)methylsulfinyl]-5-acetylbenzimidazole, and 2-(2-pyridylmethylsulfinyl)-5-acetylbenzimidazole.

7. A use according to claim 1, in which the compound is selected from 2-[(4,5-dimethyl-2-pyridyl)methylsulfinyl]-5-methoxycarbonylbenzimidazole, 2-[(3,5-dimethyl-2-pyridyl)methylsulfinyl]-5-methoxycarbonylbenzimidazole, 2-[(3,5-dimethyl-4-methoxy-2-pyridyl)methylsulfinyl]-5-methoxycarbonylbenzimidazole, 2-(2-pyridylmethylsulfinyl)benzimidazole-5-carboxylic acid ethyl ester, 2-[(4,5-dimethyl-2-pyridyl)methylsulfinyl]-5-methoxycarbonyl-6-methylbenzimidazole, 2-[(3,4-dimethyl-2-pyridyl)methylsulfinyl]-5-methoxycarbonyl-6-methylbenzimidazole, 2-[(3,5-dimethyl-2-pyridyl)methylsulfinyl]-5-methoxycarbonyl-6-methylbenzimidazole, 2-[(3,4,5-trimethyl-2-pyridyl)methylsulfinyl]-5-methoxycarbonyl-6-methylbenzimidazole, 2-[(4-methoxy-2-pyridyl)methylsulfinyl]-5-methoxycarbonyl-6-methylbenzimidazole, 2-[(4-ethoxy-2-pyridyl)methylsulfinyl]-5-methoxycarbonyl-6-methylbenzimidazole, 2-[(3-methyl-4-methoxy-2-pyridyl)methylsulfinyl]-5-methoxycarbonyl-6-methylbenzimidazole, 2-[(3,5-dimethyl-4-methoxy-2-pyridyl)methylsulfinyl]-5-methoxycarbonyl-6-methylbenzimidazole, and 2-[(4-methoxy-5-methyl-2-pyridyl)methylsulfinyl]-5-methoxycarbonyl-6-methylbenzimidazole.

8. A use according to claim 1, in which the compound is selected from 2-(2-pyridylmethylsulfinyl)-6-chlorobenzimidazole, 2-[(3,5-dimethyl-4-methoxy-2-pyridyl)methylsulfinyl]-5-chlorobenzimidazole, 2-(2-pyridylmethylsulfinyl)-5-chlorobenzimidazole, 2-[1-(2-pyridyl)propylsulfinyl]-5-chlorobenzimidazole, and 2-(2-pyridylmethylsulfinyl)-4-chlorobenzimidazole.

9. A use according to claim 1, in which the compound is selected from 2-[(4-methoxy-5-methyl-2-pyridyl)methylsulfinyl]-5-methoxybenzimidazole, 2-[(3,5-dimethyl-4-methoxy-2-pyridyl)methylsulfinyl]-b-methoxybenzimidazole, 2-(2-pyridylmethylsulfinyl)-5-methoxybenzimidazole, 2-(2-pyridylmethylsulfinyl)-5-hydroxybenzimidazole, and 2-(2-pyridylmethylsulfinyl)benzimidazole-5-carboxylic acid.

10. A use according to claim 1, in which the compound is selected from 2-[1-(2-pyridyl)ethylsulfinyl]-5-cyanobenzimidazole, 2-[1-(2-pyridyl)ethylsulfinyl]-5-trifluoromethylbenzimidazole, 2-[1-(2-pyridyl)propylsulfinyl]-5-cyanobenzimidazole, and 2-[1-(2-pyridyl)propylsulfinyl]-5-trifluoromethylbenzimidazole.

**Patentansprüche**

1. Verwendung einer Verbindung zur Herstellung eines Medikamentes für den zellschützenden Schutz gegen eine nicht-Magensäure-induzierte, nicht-traumatischinduzierte, nicht-neoplastisch Magen-Darm entzündliche Krankheit, worin die Verbindung die Formel

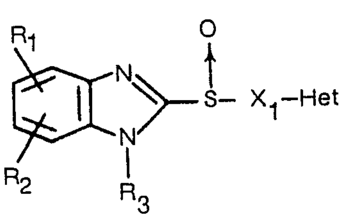

I

hat; in welchem entweder $R_1$ und $R_2$ unabhängig von H, $C_{1-4}$alkyl, F, Cl, Br, I, Cn, COOH, carboxy ($C_{1-4}$alkyl), ($C_{1-4}$alkoxy)carbonyl, ($C_{1-4}$alkoxy)carbonyl ($C_{1-4}$ alkyl), carbamoyl, carbamoyloxy, OH, $C_{1-5}$alkoxy, $C_{1-7}$hydroxyalkyl, $CF_3$ oder ($C_{1-4}$alkyl)carbonyl ausgewählt werden,

$R_3$ ist H, $C_{1-4}$alkyl, ($C_{1-3}$alkyl)carbonyl, ($C_{1-4}$alkoxy)carbonyl, carbamoyl, ($C_{1-4}$alkyl)carbamoyl, di ($C_{1-4}$alkyl)carbamoyl, ($C_{1-3}$alkyl)carbonylmethyl, ($C_{1-3}$alkoxy)carbonylmethyl oder $C_{1-4}$alkylsulfonyl;

$X_1$ ist $C_{1-4}$ alkylen; und

Het ist wahlfrei ersetztes Imidazolyl, Imidazolinyl, Benzimidazolyl, Thiazolyl, Thiazolinyl, Quinolyl, Piperidyl oder Pyridyl, worin bis zu 3 Substituenten von $C_{1-4}$alkyl, F, Cl, Br und I ausgewählt werden dürfen; oder

$R_1$ und $R_2$ werden unabhängig von H, $C_{1-4}$alkyl, F, Cl, Br, I, Methoxycarbonyl, Äthoxycarbonyl, $C_{1-4}$alkoxy und ($C_{1-4}$alkyl)carbonyl ausgewählt;

$R_3$ ist Wasserstoff; und

—$X_1$-Het wird zusammengenommen und ist Ring-ersetztes 1-phenyl-$C_{1-3}$alkyl, worin 1, 2 oder 3 Substituenten unabhängig von Methyl, Methoxy, Äthoxy, Methoxyäthoxy und Äthoxyäthoxy ausgewählt werden, mit der Bedingung, dass ein einzelner Methylsubstituent nicht existiert;

oder ein pharmakologisch verträgliches Salz desselben.

2. Verwendung nach Anspruch 1, wobei die Verbindung von
2-[(3,5-dimethyl-4-methoxy-2-pyridyl)methylsulfinyl-benzimidazol,
2-(2-pyridylmethylsulfinyl)benzimidazol,
2-[(4-chloro-2-pyridyl)methylsulfinyl]benzimidazol,
2-[(5-methyl-2-pyridyl)methylsulfinyl]benzimidazol,
2-[1-(2-pyridyl)äthylsulfinyl]benzimidazol,
2-[1-(2-pyridyl)propylsulfinyl]benzimidazol,
2-[(3-methyl-2-pyridyl)methylsulfinyl]benzimidazol,
2-[(5-äthyl-2-pyridyl)methylsulfinyl]benzimidazol, und
2-[1-(2-pyridyl)-2-methylpropylsulfinyl]benzimidazol ausgewählt wird.

3. Verwendung nach Anspruch 1, wobei die Verbindung von
2-(2-pyridylmethylsulfinyl)-N-methylbenzimidazol,
2-(2-pyridylmethylsulfinyl)-N-acetylbenzimidazol,
2-(2-pyridylmethylsulfinyl)-N-methoxycarbonylbenzimidazol,
2-(2-pyridylmethylsulfinyl)-N-carbamoylbenzimidazol,
2-(2-pyridylmethylsulfinyl)-N-methylcarbamoyl)benzimidazol,
2-(2-pyridylmethylsulfinyl)-N-acetylmethyl)benzimidazol,
2-(2-pyridylmethylsulfinyl)-N-äthoxycarbonylmethyl)benzimidazol, und
2-(2-pyridylmethylsulfinyl)-N-(methylsulfonyl)benzimidazol ausgewählt wird.

4. Verwendung nach Anspruch 1, wobei die Verbindung von
2-[(3,5-dimethyl-2-pyridyl)methylsulfinyl]-4,6-dimethyl-benzimidazol,
2-[(4-methoxy-2-pyridyl)methylsulfinyl]-4,6-dimethylbenzimidazol,
2-(2-pyridylmethylsulfinyl)-4,6-dimethylbenzimidazol,
2-(2-pyridylmethylsulfinyl)-4-methyl-6-chlorobenzimidazol, und
2-(2-pyridylmethylsulfinyl)-4-methylbenzimidazol ausgewählt wird.

5. Verwendung nach Anspruch 1, wobei die Verbindung von
2-[(3,5-dimethyl-4-methoxy-2-pyridyl)methylsulfinyl]-5-methylbenzimidazol,
2-(2-pyridylmethylsulfinyl)-5-methylbenzimidazol,
2-[(4-methyl-2-pyridyl)methylsulfinyl]-5-methylbenzimidazol,
2-[(5-methyl-2-pyridyl)methylsulfinyl]-5-methylbenzimidazol,
2-[(5-äthyl-2-pyridyl)methylsulfinyl]-5-methylbenzimidazol,
2-[1-(2-pyridyl)propylsulfinyl]-5-methylbenzimidazol,
2-(2-pyridylmethylsulfinyl)-5-äthylbenzimidazol,
2-[1-(2-pyridyl)äthylsulfinyl]-5-äthylbenzimidazol,
2-[1-(2-pyridyl)propylsulfinyl]-5-äthylbenzimidazol,
2-(2-pyridylmethylsulfinyl)-5-isopropylbenzimidazol,
2-(2-pyridylmethylsulfinyl)-5-(t-butyl)benzimidazol,
2-(2-pyridylmethylsulfinyl)-5-(n-propyl)benzimidazol,
2-[1-(2-pyridyl)äthylsulfinyl]-5,6-dimethylbenzimidazol, und
2-(2-pyridylmethylsulfinyl)-5,6-dimethylbenzimidazol ausgewählt wird.

6. Verwendung nach Anspruch 1, wobei die Verbindung von
2-[(3,4-dimethyl-2-pyridyl)methylsulfinyl]-5-acetyl-6-methylbenzimidazol,
2-[(4,5-dimethyl-2-pyridyl)methylsulfinyl]-5-acetyl-6-methylbenzimidazol,
2-[(3,5-dimethyl-2-pyridyl)methylsulfinyl]-5-acetyl-6-methylbenzimidazol,
2[(3,5-dimethyl-4-methoxy-2-pyridyl)methylsulfinyl]-5-acetylbenzimidazol, und
2-[(3,4,5-trimethyl-2-pyridyl)methylsulfinyl]-5-acetyl-6-methylbenzimidazol,
2-[(4-methoxy-2-pyridyl)methylsulfinyl]-5-acetyl-6-methylbenzimidazol,
2-[(3,5-dimethyl-4-methoxy-2-pyridyl)methylsulfinyl]-5-acetyl-6-methylbenzimidazol,
2-[(3,5-dimethyl-4-methoxy-2-pyridyl)methylsulfinyl]-5-acetylbenzimidazol, und
2-(2-pyridylmethylsulfinyl)-5-acetylbenzimidazol ausgewählt wird.

7. Verwendung nach Anspruch 1, wobei die Verbindung von
2-[(4,5-dimethyl-2-pyridyl)methylsulfinyl]-5-methoxycarbonylbenzimidazol,
2-[(3,5-dimethyl-2-pyridyl)methylsulfinyl]-5-methoxycarbonylbenzimidazol,
2-[(3,5-dimethyl-4-methoxy-2-pyridyl)methylsulfinyl]-5-methoxycarbonylbenzimidazol,
2-(2-pyridylmethylsulfinyl)benzimidazol-5-carbonsäureäthyl ester,
2-[(4,5-dimethyl-2-pyridyl)methylsulfinyl]-5-methoxycarbonyl-6-methylbenzimidazol,
2-[(3,4-dimethyl-2-pyridyl)methylsulfinyl]-5-methoxycarbonyl-6-methylbenzimidazol,
2-[(3,5-dimethyl-2-pyridyl)methylsulfinyl]-5-methoxycarbonyl-6-methylbenzimidazol,

2-[(3,4,5-trimethyl-2-pyridyl)methylsulfinyl]-5-methoxycarbonyl-6-methylbenzimidazol,
2-[(4-methoxy-2-pyridyl)methylsulfinyl]-5-methoxycarbonyl-6-methylbenzimidazol,
2-[(4-ethoxy-2-pyridyl)methylsulfinyl]-5-methoxycarbonyl-6-methylbenzimidazol,
2-[(3-methyl-4-methoxy-2-pyridyl)methylsulfinyl]-5-methoxycarbonyl-6-methylbenzimidazol, und
2-[(3,5-dimethyl-4-methoxy-2-pyridyl)methylsulfinyl]-5-methoxycarbonyl-6-methylbenzimidazol,
ausgewählt wird.
2-[(4-methoxy-5-methyl-2-pyridyl)methylsulfinyl]-5-methoxycarbonyl-6-methylbenzimidazol ausgewählt wird.

8. Verwendung nach Anspruch 1, wobei die Verbindung von
2-(2-pyridylmethylsulfinyl)-6-chlorobenzimidazol,
2-[(3,5-dimethyl-4-methoxy-2-pyridyl)methylsulfinyl]-5-chlorobenzimidazol,
2-(2-pyridylmethylsulfinyl)-5-chlorobenzimidazol,
2-[1-(2-pyridyl)propylsulfinyl]-5-chlorobenzimidazol, und
2-(2-pyridylmethylsulfinyl)-4-chlorobenzimidazol ausgewählt wird.

9. Verwendung nach Anspruch 1, wobei die Verbindung von
2-[(4-methoxy-5-methyl-2-pyridyl)methylsulfinyl]-5-methoxybenzimidazol,
2-[(3,5-dimethyl-4-methoxy-2-pyridyl)methylsulfinyl]-5-methoxybenzimidazol,
2-(2-pyridylmethylsulfinyl)-5-methoxybenzimidazol,
2-(2-pyridylmethylsulfinyl)-5-hydroxybenzimidazol, und
2-(2-pyridylmethylsulfinyl)benzimidazol-5-Carbonsäure ausgewählt wird.

10. Verwendung nach Anspruch 1, wobei die Verbindung von
2-[1-(2-pyridyl)äthylsulfinyl]-5-cyanobenzimidazol,
2-[1-(2-pyridyl)äthylsulfinyl]-5-trifluoromethyl benzimidazol,
2-[1-(2-pyridyl)propylsulfinyl]-5-cyanobenzimidazol, und
2-[1-(2-pyridyl)propylsulfinyl]-5-trifluoromethylbenzimidazol ausgewählt wird.

**Revendications**

1. Utilisation d'un composé pour la production d'un médicament destiné à la protection, par effet cytoprotecteur, d'une maladie inflammatoire gastro-intestinale non néoplastique, non induite par un traumatisme, non induite par l'acidité gastrique, dans laquelle le composé répond à la formule

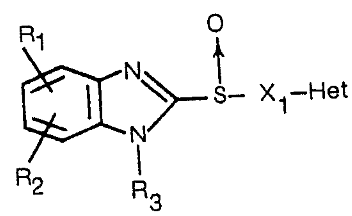

I

dans laquelle $R_1$ et $R_2$ sont choisis indépendamment entre l'hydrogène, un groupe alkyle en $C_1$ à $C_4$, F, Cl, Br, I, un groupe CN, COOH, carboxy (alkyle en $C_1$ à $C_4$), alkoxy en $C_1$ à $C_4$)carbonyle, (alkoxy en $C_1$ à $C_4$)carbonyl(alkyle en $C_1$ à $C_4$), carbamoyle, carbamoyloxy, OH, alkoxy en $C_1$ à $C_5$, hydroxyalkyle en $C_1$ à $C_7$, $CF_3$ ou (alkyle en $C_1$ à $C_4$)carbonyle;

$R_3$ représente l'hydrogène, un groupe alkyle en $C_1$ à $C_4$, (alkyle en $C_1$ à $C_3$)carbonyle, (alkoxy en $C_1$ à $C_4$)carbonyle, carbamoyle, (alkyle en $C_1$ à $C_4$)carbamoyle, di(alkyle en $C_1$ à $C_4$)carbamoyle, (alkyle en $C_1$ à $C_3$)carbonylméthyle, (alkoxy en $C_1$ à $C_3$)carbonylméthyle ou alkylsulfonyle en $C_1$ à $C_4$;

$X_1$ représente un groupe alkylène en $C_1$ à $C_4$; et

Het est un groupe, facultativement substitué, imidazolyle, imidazolinyle, benzimidazolyle, thiazolyle, thiazolinyle, quinolyle, pipéridyle ou pyridyle, dans lequel il peut exister jusqu'à 3 substituants choisis entre un groupe alkyle en $C_1$ à $C_4$, F, Cl, Br et I; ou bien

$R_1$ et $R_2$ sont choisis indépendamment entre l'hydrogène, un groupe alkyle en $C_1$ à $C_4$, F, Cl, Br, I, méthoxycarbonyle, éthoxycarbonyle, alkoxy en $C_1$ à $C_4$ et (alkyle en $C_1$ à $C_4$)carbonyle;

$R_3$ représente l'hydrogène; et

les substituants —$X_1$—Het, pris conjointement, forment un groupe phényl(alkyle en $C_1$ à $C_3$) substitué sur le noyau en position 1, dans lequel il existe 1, 2 ou 3 substituants choisis indépendamment entre un groupe méthyle, méthoxy, éthoxy, méthoxyéthoxy et éthoxyéthoxy, sous réserve qu'il n'existe pas un unique substituant méthyle;

ou d'un de ses sels pharmacologiquement acceptables.

2. Utilisation suivant la revendication 1, dans laquelle le composé est choisi entre
le 2-[(3,5-diméthyl-4-méthoxy-2-pyridyl)méthylsulfinyl]benzimidazole,
le 2-(2-pyridylméthylsulfinyl)benzimidazole,
le 2-[(4-chloro-2-pyridyl)méthylsulfinyl]benzimidazole,
le 2-[(5-méthyl-2-pyridyl)méthylsulfinyl]benzimidazole,
le 2-[1-(2-pyridyl)éthylsulfinyl]benzimidazole,
le 2-[1-(2-pyridyl)propylsulfinyl]benzimidazole,

le 2-[(3-méthyl-2-pyridyl)méthylsulfinyl]benzimidazole,
le 2-[(5-éthyl-2-pyridyl)méthylsulfinyl]benzimidazole, et
le 2-[1-(2-pyridyl)-2-méthylpropylsulfinyl]benzimidazole.

3. Utilisation suivant la revendication 1, dans laquelle le composé est choisi entre
le 2-(2-pyridylméthylsulfinyl)-N-méthylbenzimidazole,
le 2-(2-pyridylméthylsulfinyl)-N-acétylbenzimidazole,
le 2-(2-pyridylméthylsulfinyl)-N-méthoxycarbonylbenzimidazole,
le 2-(2-pyridylméthylsulfinyl)-N-carbamoylbenzimidazole,
le 2-(2-pyridylméthylsulfinyl)-N-(méthylcarbamoyl)benzimidazole,
le 2-(2-pyridylméthylsulfinyl)-N-(acétylméthyl)benzimidazole,
le 2-(2-pyridylméthylsulfinyl)-N-(éthoxycarbonylméthyl)benzimidazole, et
le 2-(2-pyridylméthylsulfinyl)-N-(méthylsulfonylbenzimidazole.

4. Utilisation suivant la revendication 1, dans laquelle le composé est choisi entre
le 2-[(3,5-diméthyl-2-pyridyl)méthylsulfinyl]-4,6-diméthylbenzimidazole,
le 2-[(4-méthoxy-2-pyridyl)méthylsulfinyl]-4,6-diméthylbenzimidazole,
le 2-(2-pyridylméthylsulfinyl)-4,6-diméthylbenzimidazole,
le 2-(2-pyridylméthylsulfinyl)-4-méthyl-6-chlorobenzimidazole, et
le 2-(2-pyridylméthylsulfinyl)-4-méthylbenzimidazole.

5. Utilisation suivant la revendication 1, dans laquelle le composé est choisi entre
le 2-[(3,5-diméthyl-4-méthoxy-2-pyridyl)méthylsulfinyl]-5-méthylbenzimidazole,
le 2-(2-pyridylméthylsulfinyl)-5-méthylbenzimidazole,
le 2-[(4-méthyl-2-pyridyl)méthylsulfinyl]-5-méthylbenzimidazole,
le 2-[(5-méthyl-2-pyridyl)méthylsulfinyl]-5-méthylbenzimidazole,
le 2-[(5-éthyl-2-pyridyl)méthylsulfinyl]-5-méthylbenzimidazole,
le 2-[1-(2-pyridyl)propylsulfinyl]-5-méthylbenzimidazole,
le 2-(2-pyridylméthylsulfinyl)-5-éthylbenzimidazole,
le 2-[1-(2-pyridyl)éthylsulfinyl]-5-éthylbenzimidazole,
le 2-[1-(2-pyridyl)propylsulfinyl]-5-éthylbenzimidazole,
le 2-(2-pyridylméthylsulfinyl)-5-isopropylbenzimidazole,
le 2-(2-pyridylméthylsulfinyl)-5-(tertio-butyl)benzimidazole,
le 2-(2-pyridylméthylsulfinyl)-5-(n-propyl)benzimidazole,
le 2-[1-(2-pyridyl)éthylsulfinyl]-5,6-diméthylbenzimidazole, et
le 2-(2-pyridylméthylsulfinyl)-5,6-diméthylbenzimidazole.

6. Utilisation suivant la revendication 1, dans laquelle le composé est choisi entre
le 2-[(3,4-diméthyl-2-pyridyl)méthylsulfinyl]-5-acétyl-6-méthylbenzimidazole,
le 2-[(4,5-diméthyl-2-pyridyl)méthylsulfinyl]-5-acétyl-6-méthylbenzimidazole,
le 2-[(3,5-diméthyl-2-pyridyl)méthylsulfinyl]-5-acétyl-6-méthylbenzimidazole,
le 2-[(3,4,5-triméthyl-2-pyridyl)méthylsulfinyl]-5-acétyl-6-méthylbenzimidazole,
le 2-[(4-méthoxy-2-pyridyl)méthylsulfinyl]-5-acétyl-6-méthylbenzimidazole,
le 2-[(3,5-diméthyl-4-méthoxy-2-pyridyl)méthylsulfinyl]-5-acétyl-6-méthylbenzimidazole,
le 2-[(3,5-diméthyl-4-méthoxy-2-pyridyl)méthylsulfinyl]-5-acétylbenzimidazole, et
le 2-(2-pyridylméthylsulfinyl)-5-acétylbenzimidazole.

7. Utilisation suivant la revendication 1, dans laquelle le composé est choisie entre
le 2-[(4,5-diméthyl-2-pyridyl)méthylsulfinyl]-5-méthoxycarbonylbenzimidazole,
le 2-[(3,5-diméthyl-2-pyridyl)méthylsulfinyl]-5-méthoxycarbonylbenzimidazole,
le 2-[(3,5-diméthyl-4-méthoxy-2-pyridyl)méthylsulfinyl]-5-méthoxycarbonylbenzimidazole,
l'ester éthylique de l'acide 2-(2-pyridylméthylsulfinyl)benzimidazole-5-carboxylique,
le 2-[(4,5-diméthyl-2-pyridyl)méthylsulfinyl]-5-méthoxycarbonyl-6-méthylbenzimidazole,
le 2-[(3,4-diméthyl-2-pyridyl)méthylsulfinyl]-5-méthoxycarbonyl-6-méthylbenzimidazole,
le 2-[(3,5-diméthyl-2-pyridyl)méthylsulfinyl]-5-méthoxycarbonyl-6-méthylbenzimidazole,
le 2-[(3,4,5-triméthyl-2-pyridyl)méthylsulfinyl]-5-méthoxycarbonyl-6-méthylbenzimidazole,
le 2-[(4-méthoxy-2-pyridyl)méthylsulfinyl]-5-méthoxycarbonyl-6-méthylbenzimidazole,
le 2-[(4-éthoxy-2-pyridyl)méthylsulfinyl]-5-méthoxycarbonyl-6-méthylbenzimidazole,
le 2-[(3-méthyl-4-méthoxy-2-pyridyl)méthylsulfinyl]-5-méthoxycarbonyl-6-méthylbenzimidazole,
le 2-[(3,5-diméthyl-4-méthoxy-2-pyridyl)méthylsulfinyl]-5-méthoxycarbonyl-6-méthylbenzimidazole, et
le 2-[(4-méthoxy-5-méthyl-2-pyridyl)méthylsulfinyl]-5-méthoxycarbonyl-6-méthylbenzimidazole.

8. Utilisation suivant la revendication 1, dans laquelle le composé est choisi entre
le 2-(2-pyridylméthylsulfinyl)-6-chlorobenzimidazole,
le 2-[(3,5-diméthyl-4-méthoxy-2-pyridyl)méthylsulfinyl]-5-chlorobenzimidazole,
le 2-(2-pyridylméthylsulfinyl)-5-chlorobenzimidazole,
le 2-[1-(2-pyridyl)propylsulfinyl]-5-chlorobenzimidazole, et
le 2-(2-pyridylméthylsulfinyl)-4-chlorobenzimidazole.

9. Utilisation suivant la revendication 1, dans laquelle le composé est choisi entre
le 2-[(4-méthoxy-5-méthyl-2-pyridyl)méthylsulfinyl]-5-méthoxybenzimidazole,
le 2-[(3,5-diméthyl-4-méthoxy-2-pyridyl)méthylsulfinyl]-5-méthoxybenzimidazole,

le 2-(2-pyridylméthylsulfinyl)-5-méthoxybenzimidazole,
le 2-(2-pyridylméthylsulfinyl)-5-hydroxybenzimidazole, et
l'acide 2-(2-pyridylméthylsulfinyl)benzimidazole-5-carboxylique.

10. Utilisation suivant la revendication 1, dans laquelle le composé est choisi entre
le 2-[1-(2-pyridyl)éthylsulfinyl]-5-cyanobenzimidazole,
le 2-[1-(2-pyridyl)éthylsulfinyl]-5-trifluorométhylbenzimidazole,
le 2-[1-(2-pyridyl)propylsulfinyl]-5-cyanobenzimidazole, et
le 2-[1-(2-pyridyl)propylsulfinyl]-5-trifluorométhylbenzimidazole.